# EUROPEAN PATENT APPLICATION

(11) **EP 3 824 809 A1**
(43) Date of publication of application: **26.05.2021**
(21) Application number: 20194770.2
(22) Date of filing: 07.09.2020
(51) Int. Cl.: A61B 5/11, A61B 5/00, A61B 5/026, A61B 5/145, A61B 5/0531, A61B 5/1171, A61B 8/08, A61B 5/16, A61B 5/0205, A01K 11/00

(54) **ANIMAL PHYSICAL ACTIVITY TRACKING DEVICE AND SYSTEM HAVING THE SAME**

(30) Priority: 19.11.2019 TW 108142032
(71) Applicant: Luxshare-ICT Co., Ltd., Taipei City 11493 (TW)
(72) Inventor: CHEN, Hsin-Nan, 11493 Taipei City (TW); HSU, Tsung-Pao, 11493 Taipei City (TW); CHIEN, Jung-Pin, 11493 Taipei City (TW); TSAI, Yao-Chun, 11493 Taipei City (TW)
(74) Representative: Friese Goeden Patentanwälte PartGmbB

(57) **Abstract**

An animal physical activity tracking device is adapted for contacting with a skin of an animal. The device comprises a substrate for contacting the skin, a sensing module disposed on the substrate, a communication module disposed on the substrate and a control module disposed on the substrate. The sensing module comprises a skin sensing module for sensing the skin to generate a dermatoglyphic signal, and a physiological sensing module for sensing the animal to generate a physiological signal. The control module outputs the dermatoglyphic signal and physiological signal through the communication module.

## Description

### Cross Reference to Related Application

This application claims the priority benefit of Taiwanese Patent Application Serial Number TW108142032, filed on November 19, 2019, the full disclosure of which is incorporated herein by reference.

### BACKGROUND

### Technical Field

The present disclosure relates to the technical field of tracking technology, and more particularly to an animal physical activity tracking device.

### Related Art

Many animals have been drastically reduced in number and are on the verge of extinction as the result of global warming, so people start engaging in animal conservation works. The information of the body condition of animals is substantial for animal conservation. By observing the physical condition and behavior of rare species or livestock, animal management can be implemented more effectively. The typical animal tracking devices are only equipped with the function of communication and positioning which could not obtain the physiological information. The physical condition of animals would change with the environment, and it is also one of the key criteria for wild release. When the real-time information of the physical condition of animals could be obtained, especially for those on contesting and livestock, the stress resistance and emotional changes of animals could be better understood.

### SUMMARY

The embodiments of the present disclosure provide an animal physical activity tracking device adapted for contacting a skin of an animal. The device comprises a substrate adapted for contacting the skin, a sensing module disposed on the substrate, a communication module disposed on the substrate, and a control module disposed on the substrate. The sensing module comprises a skin sensing module for sensing the skin to generate a dermatoglyphic signal, and a physiological sensing module for sensing the animal to generate a physiological signal. The control module outputs the dermatoglyphic signal and the physiological signal through the communication module.

According to the embodiments, the skin sensing module comprises an ultrasonic sensor for sensing the skin to generate the dermatoglyphic signal.

According to the embodiments, the skin sensing module comprises an optical sensor for sensing the skin to generate the dermatoglyphic signal.

According to the embodiments, the physiological sensing module comprises an ultrasonic sensor for sensing the changes in the size of a pore on the skin and the flow rate of a vascular fluid of the animal to generate the physiological signal.

According to the embodiments, the physiological sensing module comprises a cortisol sensor for sensing a cortisol concentration of the animal to generate the physiological signal.

According to the embodiments, the physiological sensing module comprises a skin electric conductivity sensor for sensing a skin electric conductivity coefficient of the animal to generate the physiological signal.

According to the embodiments, the animal physical activity tracking device further comprises a positioning module disposed on the substrate generating a positioning signal based on a position of the animal, wherein the control module outputs the positioning signal through the communication module.

According to the embodiments, an animal physical activity tracking system comprises an animal physical activity tracking device and a server. The animal physical activity tracking device comprises a substrate adapted for contacting the skin, a sensing module disposed on the substrate, a communication module disposed on the substrate, and a control module disposed on the substrate. The sensing module comprises a skin sensing module for sensing the skin to generate a dermatoglyphic signal, and a physiological sensing module for sensing the animal to generate a physiological signal. The control module outputs the dermatoglyphic signal and the physiological signal through the communication module. The server comprises a receiver for receiving the dermatoglyphic signal and the physiological signal, a controller for processing the dermatoglyphic signal and the physiological signal to obtain an individual identifier and a physiological condition corresponding to the animal, and a storage for storing the individual identifier and the physiological condition.

Therefore, according to the embodiments, the animal physical activity tracking device is configured to be disposed on animals by the substrate such that the condition of physical activity of the animals could be detected at any time when the animals move or are doing something. The physical activity signals detected by the sensor modules correspond to the physiological changes of the individuals of the animals, such that collected data could be avoided from being obfuscated. It can also classify different species of animals to facilitate data collection and further applications and can obtain information of the changes in the physical activity of animals through a variety of sensors, such as ultrasonic sensors, skin electric conductivity sensors, and cortisol sensors. In addition, when receiving the physiological information transmitted by the animal physical activity tracking device, the server could monitor the physical condition and other related data of animals through the method of analysis and statistics.

It should be understood, however, that this summary may not contain all aspects and embodiments of the present invention, that this summary is not meant to be limiting or restrictive in any manner, and that the invention as disclosed herein will be understood by one of ordinary skill in the art to encompass obvious improvements and modifications thereto.

### BRIEF DESCRIPTION OF THE DRAWINGS

The features of the exemplary embodiments believed to be novel and the elements and/or the steps characteristic of the exemplary embodiments are set forth with particularity in the appended claims. The Figures are for illustration purposes only and are not drawn to scale. The exemplary embodiments, both as to organization and method of operation, may best be understood by reference to the detailed description which follows taken in conjunction with the accompanying drawings in which:
FIG. 1 is a block diagram of an animal physical activity tracking device of the embodiments of the present disclosure;
FIG. 2 is a block diagram of a skin sensing module of the embodiments of the present disclosure;
FIG. 3 is a block diagram of a physiological sensing module of the embodiments of the present disclosure; and
FIG. 4 is a block diagram of an animal physical activity tracking system of the embodiments of the present disclosure.

### DETAILED DESCRIPTION OF THE EMBODIMENTS

The present invention will now be described more fully hereinafter with reference to the accompanying drawings, in which exemplary embodiments of the invention are shown. This present invention may, however, be embodied in many different forms and should not be construed as limited to the embodiments set forth herein. Rather, these embodiments are provided so that this present invention will be thorough and complete, and will fully convey the scope of the present invention to those skilled in the art.

Certain terms are used throughout the description and following claims to refer to particular components. As one skilled in the art will appreciate, manufacturers may refer to a component by different names. This document does not intend to distinguish between components that differ in name but function. In the following description and in the claims, the terms "include/including" and "comprise/comprising" are used in an open-ended fashion, and thus should be interpreted as "including but not limited to". "Substantial/substantially" means, within an acceptable error range, the person skilled in the art may solve the technical problem in a certain error range to achieve the basic technical effect.

The following description is of the best-contemplated mode of carrying out the invention. This description is made for the purpose of illustration of the general principles of the invention and should not be taken in a limiting sense. The scope of the invention is best determined by reference to the appended claims.

Moreover, the terms "include", "contain", and any variation thereof are intended to cover a non-exclusive inclusion. Therefore, a process, method, object, or device that comprises a series of elements not only include these elements, but also comprises other elements not specified expressly, or may include inherent elements of the process, method, object, or device. If no more limitations are made, an element limited by "include a/an ..." does not exclude other same elements existing in the process, the method, the article, or the device which comprises the element.

In the following embodiment, the same reference numerals are used to refer to the same or similar elements throughout the invention.

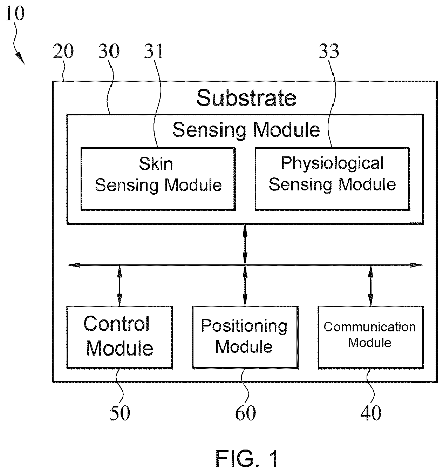

FIG. 1 is a block diagram of an animal physical activity tracking device of the embodiments of the present disclosure. The animal physical activity tracking device 10 is adapted for contacting a skin of an animal. The animal physical activity tracking device 10 comprises a substrate 20 adapted for contacting the skin, a sensing module 30 disposed on the substrate 20, a communication module 40 disposed on the substrate 20, and a control module 50 disposed on the substrate 20. The sensing module 30 comprises a skin sensing module 31 for sensing the skin to generate a dermatoglyphic signal and a physiological sensing module 33 for sensing the animal to generate a physiological signal. The control module 50 outputs the dermatoglyphic signal and the physiological signal through the communication module 40.

The animal physical activity tracking device 10 is adapted for tracking the physical activity of an animal, such as but not limited to the physiological condition of the animal. The physiological condition could be, but not limited to, the changes in pore size, vascular fluid flow rate, skin electric conductivity coefficient, and/or cortisol concentration of the animal. In some embodiments, the animal activity tracking device 10 can transmit the positioning information of the animal. In the embodiments of the present disclosure, the animal activity tracking device 10 can transmit an identification signal or an identifier of an animal corresponding to the animal activity tracking device 10. The identification signal or the identifier may be, but not limited to, the dermatoglyphic signal of the animal.

The substrate 20 is adapted for contacting the skin of an animal. In the embodiments of the present disclosure, the substrate 20 is a film or an electronic skin adapted for being attached to the skin of an animal. With excellent adhesion, the substrate 20 could hardly fall off when the animal moves, shakes, or wobbles. For example, the substrate 20 can be attached to the neck or back of an animal; alternatively, the substrate 20 could be a wearable device or a part of a wearable device, such as ankle bracelet, collar, or chest strap, secured onto the animal. The substrate 20 can also contact the skin of the animal. For example, the substrate 20 could be configured into a form of ankle bracelet or could be disposed on an ankle bracelet. The ankle bracket can be put on the ankle or leg of a bird, having the substrate 20 contacting the skin of the ankle or leg of the bird.

The communication module 40 is disposed on the substrate 20. The communication module 40 could be a wireless communication module 40 adopting wireless transmission techniques such as, but not limited to, Bluetooth, wireless hotspot WiFi, or mobile communication. In the embodiments of the present disclosure, the communication module 40 could be a wired communication module 40 adopting wired transmission techniques such as, but not limited to, Ethernet or Universal Serial Bus (USB).

The control module 50 is disposed on the substrate 20. The control module 50 could be, but is not limited to, a central processing unit (CPU), a microprocessor, an application-specific integrated circuit (ASIC), a system-on-a-chip (SOC), etc. The control module 50 is electrically connected to the sensing module 30 and receives a dermatoglyphic signal and a physiological signal from the sensing module 30. In the embodiments of the present disclosure, the control module 50 can be electrically connected to the sensing module 30 through a bus system. The control module 50 outputs the dermatoglyphic signal and the physiological signal to an external device such as, but not limited to, servers, microprocessors, computers, or cloud storage service through the communication module 40 (described hereafter). In the embodiments of the present disclosure, the control module 50 is electrically connected to the communication module 40 through a bus system. In the embodiments of the present disclosure, the dermatoglyphic signal could be, but not limited to, an image signal, a serial signal, etc. In the embodiments of the present disclosure, the physiological signals could be, but not limited to, brain wave signals of an animal measured by a brain wave sensor of the physiological sensing module 33 when the animal physical activity tracking device 10 contacts the head of an animal, muscle contraction signals of an animal measured by a myoelectric sensor of the physiological sensing module 33 when the animal physical activity tracking device 10 contacts an animal, electro-cardiac signals of an animal measured by an electro-cardio sensor of the physiological sensing module 33 when the animal physical activity tracking device 10 contacts the skin close to the heart of an animal, etc.

In the embodiments of the present disclosure, the animal physical activity tracking device 10 further comprises a positioning module 60 disposed on the substrate 20 and the positioning module 60 generates a positioning signal according to a position of an animal The control module 50 outputs the positioning signal through the communication module 40. The positioning module 60 could be, but not limited to, a satellite positioning module or the global positioning system (GPS) module.

The sensing module 30 is disposed on the substrate 20. The sensing module 30 comprises a skin sensing module 31 for sensing the skin to generate a dermatoglyphic signal and a physiological sensing module 33 for sensing the animal to generate a physiological signal. In the embodiments of the present disclosure, the sensing module 30 is an ultrasonic module, comprising a skin sensing module 31 and a physiological sensing module 33. The ultrasonic module could be, but not limited to, a sound ranging module. In the embodiments of the present disclosure, the ultrasonic module emits ultrasonic waves to detect the dermatoglyph and skin thickness of an animal to generate the dermatoglyphic signal by a distance measuring function based on the time-of-flight principle of ultrasonic. In the embodiments of the present disclosure, the ultrasonic module emits ultrasonic waves and uses the Doppler effect of waves to calculate the speed of an object. Based on this, the changes in pore size of animal skin and vascular fluid flow rate can be obtained to generate the physiological signals.

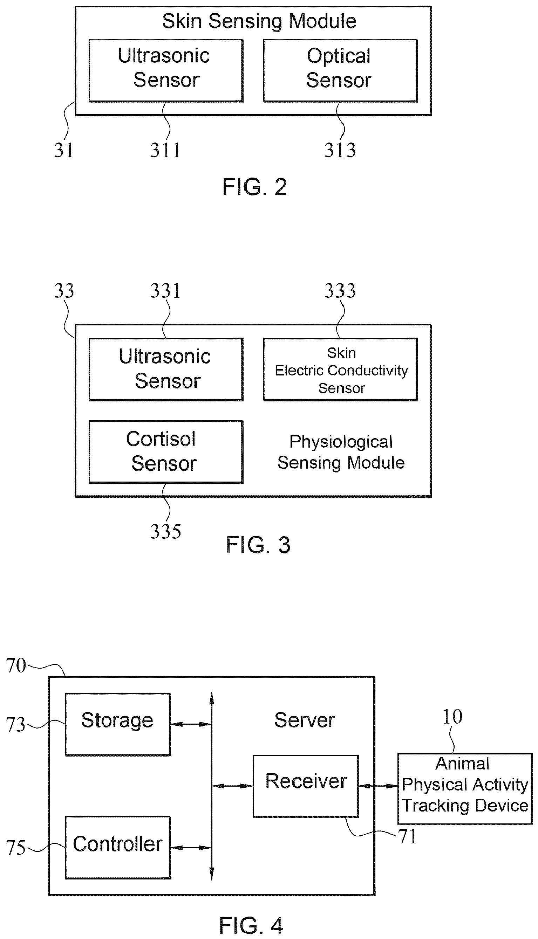

FIG. 2 is a block diagram of a skin sensing module 31 of the embodiments of the present disclosure. The sensing module 30 is disposed on the substrate 20. The sensing module 30 comprises a skin sensing module 31 for sensing the skin to generate a dermatoglyphic signal and a physiological sensing module 33 for sensing the animal to generate a physiological signal. In the embodiments of the present disclosure, the skin sensor module 31 comprises an ultrasonic sensor 311 and an optical sensor 313. The ultrasonic sensor 311 and the optical sensor 313 are used to sense the skin to generate the dermatoglyphic signal.

In the embodiments of the present disclosure, the ultrasonic sensor 311 detects the skin to generate the dermatoglyphic signal. The ultrasonic sensor 311 could be, but not limited to, an ultrasonic distance measuring module. The ultrasonic sensor 311 emits ultrasonic waves to detect the dermatoglyph and skin thickness of an animal to generate the dermatoglyphic signal by a distance measuring function based on the time-of-flight principle of ultrasonic. and to penetrate the outer skin layer of an animal, such as hair and skin, to The ultrasonic sensor 311 has capability to measure the dermatoglyph under the animal hair to generate the dermatoglyphic signal by the ultrasonic wave penetrating through the outer skin layer of an animal, such as hair and skin. In the embodiments of the present disclosure, the ultrasonic sensor 311 can detect the dermatoglyph of animal skin from different angles to generate multiple two-dimensional ultrasonic images and can synthesize the two-dimensional images into a three-dimensional stereoscopic image in an image synthesis technology by an external computer. In the embodiments of the present disclosure, the ultrasonic sensor 311 could detect three types of information by ultrasound and generates the dermatoglyphic signals based on the three types of information. One of the tree types of information is the dermatoglyph and wrinkles of animal skin; another is the node where an individual dermatoglyph ends and branches, and the last one is the position where the pores are and where the wrinkles start.

In the embodiments of the present disclosure, the optical sensor 313 is adopted to detect the skin to generate the dermatoglyphic signal. The optical sensor 313 could be, but not limited to, an optical scanner or a module having a light source, a prism, a photosensitive component, etc. By refraction and reflection of light, the optical sensor 313 can detect dermatoglyph and color of animal skin to generate the dermatoglyphic signal. In a case of the module comprising a light source, a prism and a photosensitive component, the light is emitted by the light source towards the prism and passes through the prism to project onto the skin of an animal. The angles at which the emitted light is refracted on the uneven pattern on the skin surface of an animal varies, and the brightness of every reflected light also varies. As a result, the reflected light projected on the photosensitive element through the prism presents a black-and-white or multi-grayscale image.

In this embodiment, as shown in FIG. 2, the dermatoglyphic signal may be a signal detected by the integration of the ultrasonic sensor 311 and the optical sensor 313. In other embodiments, the skin sensor module 31 could comprise only the ultrasonic sensor 311 or only the optical sensor 313, and the dermatoglyphic signal is a signal sensed by the ultrasonic sensor 311 or the optical sensor 313.

FIG. 3 is a block diagram of a physiological sensing module of the embodiments of the present disclosure. The physiological sensing module 33 comprises an ultrasonic sensor 331, a skin electric conductivity sensor 333, and a cortisol sensor 335. The ultrasonic sensor 331 detects the changes in the size of a pore on the skin and the flow rate of a vascular fluid of the animal to generate the physiological signal. The skin electric conductivity sensor 333 detects a skin electric conductivity coefficient of the animal to generate the physiological signal. The cortisol sensor 335 detects a cortisol concentration of the animal to generate the physiological signal.

In the embodiments of the present disclosure, the ultrasonic sensor 331 detects the changes of the size of a pore on the skin and the flow rate of a vascular fluid of the animal to generate the physiological signal. Since the ultrasonic waves are penetrative and would not be affected by light, the physiological signal can be generated by analyzing the ultrasonic waves emitted from the ultrasonic sensor 331 with the Doppler effect. The frequency of the reflected echo of the ultrasonic waves reflected by an object changes when the object moves. When an object moves toward the receiver of the ultrasonic sensor 331, the frequency increases. On the other hand, when the object is moving away, the frequency will decrease. As the frequency changes, the ultrasonic sensor 331 could calculate the moving speed of the object, and based on this, the changes of the pore size of the animal skin and the flow rate of the vascular fluid of an animal can be further obtained to generate physiological signals. In the embodiments of the present disclosure, the ultrasonic sensor 331 could detect changes of the pore size and the flow rate of vascular fluid from different angles to generate multiple two-dimensional ultrasonic images and to synthesize the two-dimensional images to obtain a three-dimensional image in a synthesis technology by an external computer. Based on the physiological signals representing the changes of the pore size of skin or the vascular fluid flow rate, the system could determine and analyze the physiological and emotional conditions of animals, such as the physical conditions, the responses to the environment, and the stress or the emotional changes affected by animals.

In the embodiments of the present disclosure, the skin electric conductivity sensor 333 detects a skin electric conductivity coefficient of the animal to generate the physiological signal. The skin electric conductivity sensor 333 can contact the skin of an animal by two electrodes to measure the skin current and obtain a skin electric conductivity coefficient. The skin electric conductivity coefficient of an animal would change with the external environment and the emotional condition of the animal. The skin electric conductivity sensor 333 generates physiological signals with the skin electric conductivity coefficient and further obtains the information of the physiological and emotional conditions of animals.

In the embodiments of the present disclosure, the cortisol sensor detects a cortisol concentration of the animal to generate the physiological signal. When animals experience stress, the adrenal glands would release the cortisol. The cortisol sensor 335 could generate the physiological signal by detecting the cortisol concentration of the sweat from the skin of an animal, so as to further obtain the information of physiological and emotional conditions of animals.

In this embodiment, as shown in FIG. 3, the physiological signal could be a signal integrated from the signals of the ultrasonic sensor 331, the skin conductance sensor 333, and the cortisol sensor 335. In other embodiments, the physiological sensing module 33 may comprise only one or two of the ultrasonic sensor 331, the skin electric conductivity sensor 333 and the cortisol sensor 335, and the physiological signal is a signal detected by the one or two of the ultrasonic sensor 331, the skin electric conductivity sensor 333 and the cortisol sensor 335.

FIG. 4 is a block diagram of an animal physical activity tracking system of the embodiments of the present disclosure. The animal physical activity tracking system comprises an animal physical activity tracking device 10 and a server 70. The animal physical activity tracking device 10 comprises a substrate 20 suitable for contacting the skin of an animal, a sensing module 30 disposed on the substrate 20, a communication module 40 disposed on the substrate 20, and a control module 50 disposed on the substrate 20 outputting the dermatoglyphic signal and the physiological signal through the communication module 40. The sensing module 30 comprises a skin sensing module 31 for sensing the skin to generate a dermatoglyphic signal and a physiological sensing module 33 for sensing the animal to generate a physiological signal. The server 70 comprises a receiver 71 for receiving the dermatoglyphic signal and the physiological signal, a storage 73 for storing the individual identifier and the physiological condition, and a controller 75 for processing the dermatoglyphic signal and the physiological signal to obtain an individual identifier and a physiological condition corresponding to the animal.

The server 70 could be, but not limited to, a website, a cloud storage device, a management platform, etc. The receiver 71 could be, but not limited to, a communication interface or other devices having a communication receiving function. The storage 73 could be, but not limited to, an electronically erasable programmable read-only memory (EEPROM), flash memory, etc. The controller 75 could be, but not limited to, a microprocessor, a microcontroller, a digital signal processor, a microcomputer, a central processing unit, etc.

In the embodiments of the present disclosure, the controller 75 comprises an arithmetic module for processing the dermatoglyphic signal, the physiological signal, the individual identifier and a physiological condition of the corresponding animal. The individual identifier could be, but not limited to, images and sequences formed by the dermatoglyph, gloss, hair, and thickness of the skin of an individual animal, or one or a combination of other characteristics that can be used to identify individual animals. The dermatoglyphic characteristics of an individual of animals would be unique and can be distinct from any other individuals even within the same species, as the fingerprint characteristics of humans. Therefore, the dermatoglyphic signal could be an identifier used to identify and track animals. The physiological state could be, but not limited to, heart rate, nervousness level, or other indicators capable of determining the physiological or emotional condition of the animal.

In the embodiments of the present disclosure, the server 70 can cooperate with a plurality of animal physical activity tracking devices 10. One server 70 could correspond to a plurality of animal physical activity tracking devices 10, and each animal physical activity tracking device 10 corresponds to one animal. Animals are identified by the identifier (ID). The animal physical activity tracking devices 10 could automatically transmit physiological and positional information at a predetermined frequency. In addition to the real-time monitoring of the physiological condition of animals, the server 70 can also analyze the physiological signals of a particular animal when the collected information is sufficient to obtain a short-term or long-term health or activity condition of the animal. In the embodiments of the present disclosure, the server 70 classifies the animal species and analyzes the physiological signals of the specific animal in a specific period of time to understand the physical condition of the specific animal in a specific period of time. In the embodiments of the present disclosure, the server 70 can also analyze the physiological signals of all the animals equipped with the animal physical activity tracking device 10 in the same area to understand the physiological signals of the animals in the area in a specific period of time. In the embodiments of the present disclosure, the server 70 can also combine the physiological signals of animals in a specific area with weather information (such as temperature, humidity, rainfall, and/or wind) for correlation analysis and etc.

In summary, in the embodiments of the present disclosure, the animal physical activity tracking device is configured to be disposed on animals by the substrate such that the condition of physical activity of the animals could be detected at any time when the animals move or are doing something. The physical activity signals detected by the sensor modules correspond to the physiological changes of the individuals of the animals, such that collected data could be avoided from being obfuscated. It can also classify different species of animals to facilitate data collection and further applications and can obtain information of the changes in the physical activity of animals through a variety of sensors, such as ultrasonic sensors, skin electric conductivity sensors, and cortisol sensors. In addition, when receiving the physiological information transmitted by the animal physical activity tracking device, the server could monitor the physical condition and other related data of animals through the method of analysis and statistics.

It is to be understood that the term "comprises", "comprising", or any other variants thereof, is intended to encompass a non-exclusive inclusion, such that a process, method, article, or device of a series of elements not only include those elements but also comprises other elements that are not explicitly listed, or elements that are inherent to such a process, method, article, or device. An element defined by the phrase "comprising a ..." does not exclude the presence of the same element in the process, method, article, or device that comprises the element.

Although the present invention has been explained in relation to its preferred embodiment, it does not intend to limit the present invention. It will be apparent to those skilled in the art having regard to this present invention that other modifications of the exemplary embodiments beyond those embodiments specifically described here may be made without departing from the spirit of the invention. Accordingly, such modifications are considered within the scope of the invention as limited solely by the appended claims.

## Claims

1. Animal physical activity tracking device (10) adapted for contacting a skin of an animal, comprising:
a substrate (20) adapted for contacting the skin;
**characterised in that** the animal physical activity tracking (10) further comprises:
a sensing module (30) disposed on the substrate (20), comprising a skin sensing module (31) for sensing the skin to generate a dermatoglyphic signal and a physiological sensing module (33) for sensing the animal to generate a physiological signal;
a communication module (40) disposed on the substrate (20); and
a control module (50) disposed on the substrate (20) outputting the dermatoglyphic signal and the physiological signal through the communication module (40).

2. Animal physical activity tracking device (10) according to claim 1, **characterised in that** the skin sensing module (31) comprises an ultrasonic sensor (311) for sensing the skin to generate the dermatoglyphic signal.

3. Animal physical activity tracking device (10) according to any of the preceding claims, **characterised in that** the skin sensing module (31) further comprises an optical sensor (313) for sensing the skin to generate the dermatoglyphic signal.

4. Animal physical activity tracking device (10) according to any of the preceding claims, **characterised in that** the physiological sensing module (33) comprises an ultrasonic sensor (311) for sensing the changes of the size of a pore on the skin and the flow rate of a vascular fluid of the animal to generate the physiological signal.

5. Animal physical activity tracking device (10) according to any of the preceding claims, **characterised in that** the physiological sensing module (33) further comprises a skin electric conductivity sensor (333) for sensing a skin electric conductivity coefficient of the animal to generate the physiological signal.

6. Animal physical activity tracking device (10) according to any of the preceding claims, **characterised in that** the physiological sensing module (33) further comprises a cortisol sensor (335) for sensing a cortisol concentration of the animal to generate the physiological signal.

7. Animal physical activity tracking device (10) according to any of the preceding claims, **characterised in that** the physiological sensing module (33) comprises:
an ultrasonic sensor (331) for sensing the changes of the size of a pore on the skin and the flow rate of a vascular fluid of the animal to generate the physiological signal;
a skin electric conductivity sensor (333) for sensing a skin electric conductivity coefficient of the animal to generate the physiological signal; and
a cortisol sensor (335) for sensing a cortisol concentration of the animal to generate the physiological signal.

8. Animal physical activity tracking device (10) according to any of the preceding claims, further comprising a positioning module (60) disposed on the substrate (20) and generating a positioning signal based on a position of the animal, **characterised in that** the control module (50) outputs the positioning signal through the communication module (40).

9. Animal physical activity tracking system, comprising:
an animal physical activity tracking device (10) adapted for contacting a skin of an animal and a server (70) communicated with the animal physical activity tracking device (10), the animal physical activity tracking device (10) comprising:
a substrate (20) adapted for contacting the skin;
**characterised in that** the animal physical activity tracking further comprises:
a sensing module (30) disposed on the substrate (20), comprising a skin sensing module (31) for sensing the skin to generate a dermatoglyphic signal and a physiological sensing module (33) for sensing the animal to generate a physiological signal;
a communication module (40) disposed on the substrate (20); and
a control module (50) disposed on the substrate (20) outputting the dermatoglyphic signal and the physiological signal through the communication module (40); and
the server (70) comprises:
a receiver (71) for receiving the dermatoglyphic signal and the physiological signal;
a controller (75) for processing the dermatoglyphic signal and the physiological signal to obtain an individual identifier and a physiological condition corresponding to the animal; and
a storage (73) for storing the individual identifier and the physiological condition.

10. Animal physical activity tracking system according to any of the preceding claims, **characterised in that** the skin sensing module (31) comprises an ultrasonic sensor (331) for sensing the skin to generate the dermatoglyphic signal.

11. Animal physical activity tracking system according to any of the preceding claims, **characterised in that** the skin sensing module (31) further comprises an optical sensor (313) for sensing the skin to generate the dermatoglyphic signal.

12. Animal physical activity tracking system according to any of the preceding claims, **characterised in that** the physiological sensing module (33) comprises an ultrasonic sensor (331) for sensing the changes of the size of a pore on the skin and the flow rate of a vascular fluid of the animal to generate the physiological signal.

13. Animal physical activity tracking system according to any of the preceding claims, **characterised in that** the physiological sensing module (33) further comprises:
a skin electric conductivity sensor (333) for sensing a skin electric conductivity coefficient of the animal to generate the physiological signal; and
a cortisol sensor (335) for sensing a cortisol concentration of the animal to generate the physiological signal.

14. Animal physical activity tracking system according to any of the preceding claims, **characterised in that** the animal physical activity tracking device (10) further comprises a positioning module (60) disposed on the substrate (20) and generating a positioning signal based on a position of the animal, and the control module (50) outputs the positioning signal through the communication module (40) to the server (70).
